**Europäisches Patentamt**

**European Patent Office**

**Office européen des brevets**

(19)

(11) Veröffentlichungsnummer: **0 043 483**
**B1**

(12) ## EUROPÄISCHE PATENTSCHRIFT

(45) Veröffentlichungstag der Patentschrift:
20.06.84

(51) Int. Cl.³: **G 01 N 33/72**

(21) Anmeldenummer: **81104780.2**

(22) Anmeldetag: **22.06.81**

(54) **Kunststoff-Einweg-Messküvette enthaltend eine Reaktionslösung und deren Verwendung zur Bestimmung von Hämoglobin.**

(30) Priorität: **01.07.80 DE 3024835**

(43) Veröffentlichungstag der Anmeldung:
**13.01.82 Patentblatt 82/2**

(45) Bekanntmachung des Hinweises auf die Patenterteilung:
**20.06.84 Patentblatt 84/25**

(84) Benannte Vertragsstaaten:
**AT BE CH DE FR GB IT LI NL SE**

(56) Entgegenhaltungen:
**DE - A - 1 932 749**
**DE - A - 1 933 689**
**DE - A - 2 149 763**
**DE - A - 2 250 886**
**DE - A - 2 422 260**
**DE - A - 2 501 855**
**DE - A - 2 721 942**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Frank, Georg, Dr., Am Eckbusch 55b, D-5600 Wuppertal (DE)**
Erfinder: **Wehling, Klaus, Dr., Im Johannistal 18, D-5600 Wuppertal (DE)**

## Beschreibung

Die Erfindung betrifft eine Kunstoff-Einweg-Messküvette für die Hämoglobinbestimmung enthaltend eine Reaktionslösung aus Kaliumcyanid, Kaliumhexacyanoferrat (III), einer Puffersubstanz zur Einstellung des pH-Wertes und einem Detergenz sowie deren Verwendung. Dieses Reagenz basiert auf der international anerkannten ICSH-Referenzmethode (1).

Als genauestes Verfahren zur Hämoglobinbestimmung wird heute die Hämiglobincyanidmethode angesehen (2, 3, 4). Neben den üblichen Testpackungen werden für die Hämoglobinbestimmung auch Fertigtests angeboten, die es dem Benutzer erlauben, ohne selbst das Reagenz ansetzen zu müssen, die Bestimmung schnell und einfach durchzuführen. Bei diesen Fertigtests, auch Monotest, Eintest oder Einglastest genannt, ist das Hämoglobinreagenz in Glasfläschchen abgefüllt und muss zur Messung noch in Küvetten umgefüllt werden.

Teilweise wird das Reagenz auch in Rundküvetten angeboten, in denen die Hämoglobinbestimmung direkt durchgeführt werden kann. Diese Rundküvetten sind auch aus Glas gefertig.

In neuerer Zeit sind verschliessbare Küvetten (DT-A Nr. 2422260) bekannt geworden, die als Kunstoff-Einweg-Messküvetten (vorzugsweise aus Polystyrol) ausgebildet sind und mit einem Kunststoffdeckel luftdicht verschlossen werden.

Dabei wird bei der Massenfertigung der Deckel durch Verschweissen, z.B. mittels Ultraschall, mit der Küvette verbunden. Der Deckel besitzt eine Sollbruchstelle, die man mittels eines Zapfens aufbrechen kann. Durch die so erzeugte Öffnung im Küvettendeckel kann die zu analysierende Probe in die Küvette eingebracht werden.

Die in der DE-A Nr. 2422260 beschriebenen Küvetten erfüllen bereits weitgehend die Anforderungen an einen Fertigtest. Der Anwender muss weder Reagenzien auflösen, noch verschiedene Lösungen exakt mischen, noch das fertige Reagenzprobegemisch in ein Messgefäss überführen. Er muss lediglich die zu analysierende Probe exakt dosiert in die Küvette einbringen, dies kann z.B. einfach durch volumenkalibrierte Glaskapillaren erfolgen, und die Küvette in das Messgerät (Photometer) überführen.

Dadurch werden mögliche Fehlerquellen (Pipettieren) von vornherein ausgeschaltet und der Analysenvorgang so vereinfacht, dass selbst ungeschultes Personal die Analyse richtig durchführen kann.

Die Verwendung von Einweg-Messküvetten bringt jedoch Probleme mit sich. Glas scheidet als Material für Einweg-Messküvetten aus, da Glasküvetten in der für photometrische Messungen benötigten Qualität viel zu teuer sind.

Die Verwendung von Kunststoffen, z.B. Polystyrol, erlaubt zwar eine preisgünstige Massenherstellung von Einweg-Messküvetten, bringt jedoch häufig Probleme hinsichtlich der Haltbarkeit der Reagenzien mit sich.

So ist Polystyrol für viele Gase durchlässig. Das wirkt sich z.B. negativ auf die Haltbarkeit des Reagenzes zur Bestimmung von Hämoglobin als Hämiglobincyanid aus. Dieses Reagenz enthält, wie oben erwähnt, neben einer Puffersubstanz und einem Detergenz zur Hämolyse der Erythrozyten noch Kaliumhexacyanoferrat (III), das Hämoglobin zu Hämiglobin oxidiert und KCN das Hämiglobin in Hämiglobincyanid überführt. Das Hämiglobincyanid wird anschliessend photometrisch bestimmt.

Das in dem Reagenz enthaltene KCN steht mit HCN im Gasraum über der Flüssigkeit im Gleichgewicht. HCN kann jedoch durch die Polystyrolwand der Küvette diffundieren, so dass der CN-Gehalt des Reagenzes mit einer Halbwertzeit von ca. 4 Wochen abnimmt. Dadurch ergibt sich eine für den Verkauf nicht ausreichende Haltbarkeit des Reagenzes.

In der DE-B Nr. 2721942 wird ein Verfahren beschrieben, das diese Haltbarkeitsprobleme überwindet.

Bei dem angegebenen Verfahren wird ein Reaktionspartner — im Falle der Hämoglobinbestimmung das KCN — in fester Form auf die innere Oberfläche der zur Probendosierung verwendeten Glaskapillare aufgebracht.

Durch die Verlagerung der instabilen Komponente des KCN aus der Reaktionslösung in die zur Probendosierung verwendete Kapillare werden lange Haltbarkeiten (18 bis 24 Monate) erzielt.

Nachteilig an dem beschriebenen Verfahren ist der Umstand, dass die Herstellung der mit KCN auf der inneren Oberfläche beschichteten Kapillaren mit grosser Genauigkeit erfolgen muss und entsprechend teuer ist.

Aufgabe der vorliegenden Erfindung war es nun, ein preiswerteres Verfahren zu finden, um die Konkurrenzfähigkeit der Hämoglobin-Fertig-Küvette zu sichern. Dabei musste die angestrebte Lösung qualitativ gleichwertig sein. Dies gilt besonders hinsichtlich der Präzision und Richtigkeit der Hämoglobinbestimmung als auch hinsichtlich der benötigten Reaktionszeit.

Es wurden schon verschiedene Versuche unternommen, ein in Kunststoff-Fertig-Küvetten stabiles Hämoglobinreagenz herzustellen.

So wurde z.B. der pH-Wert des Reagenzes in den alkalischen Bereich verschoben, um das Cyanid im Reagenz zu stabilisieren. Dadurch werden auch bessere Haltbarkeiten erzielt. Ein entscheidender Nachteil des Verfahrens ist jedoch darin zu sehen, dass die Reaktionszeit erheblich verlängert wird. So ist die Überführung des Hämoglobins in Hämiglobincyanid mit einem Reagenz, wie z.B in der Vornorm DIN 58 931 Blatt 1 vom November 1970 beschrieben, nach einer Reaktionszeit von 3 min abgeschlossen. Verschiebt man nun den pH-Wert des Reagenzes, ohne weitere Bestandteile zu verändern, von pH 7,2 auf pH 8,2, so verlängert sich diese Reaktionszeit auf 12 bis 15 min. Dies ist z.B. für Notfallbestimmungen ein wesentlicher Nachteil.

Andere Hersteller versuchten, durch Erhöhung der Phospatpufferkonzentration von 2,5 mmol auf 10 mmol ein in Kunststoffküvetten stabiles Reagenz zu erhalten. Aber auch diese Versuche führten zu keinem Erfolg.

Ein weiterer Nachteil der Erhöhung der Phosphatpufferkonzentration ist die Tatsache, dass dadurch die Stabilität des K3 [Fe (CN)6]-Komplexes negativ beeinflusst wird. Versuche, eine ausreichende Haltbarkeit des Hämoglobinreagenzes in Kunststoffküvetten durch Überdosieren des KCN zu erreichen, scheitern aus zwei Gründen. Einerseits ist die Halbwertzeit des KCN in Kunststoff-Fertig-Küvetten mit ca. 4 Wochen sehr kurz, so dass für eine Haltbarkeit von einem Jahr die 32-fache Sollkonzentration (das wären bei einer Sollkonzentration von 65,12 mg KCN/l Reagenz ca. 2080 mg) eingesetzt werden müsste. Andererseits wird durch hohe KCN-Konzentrationen der K3 [Fe (CN)6]-Komplex in der Haltbarkeit beeinträchtigt. Während z.B. bei einer Ausgangskonzentration von 65 mg KCN/l Reagenz die Konzentration des K3 [Fe (CN)6] über ein Jahr mit ca. 200 mg/l konstant bleibt, nimmt sie bei einer KCN-Ausgangskonzentration von 860 mg/l innerhalb von 18 Wochen von 200 mg/l auf 130 mg/l ab und liegt damit in einem Bereich, in dem bei der Hämoglobinbestimmung deutliche Abweichungen vom Sollwert auftreten.

Verschiedentlich wurde auch versucht, durch Zugabe von Konservierungsmitteln oder von Stabilisierungsmitteln, eine bessere Haltbarkeit des Hämoglobinreagenzes zu erreichen. Eingesetzt wurden hierfür Substanzen wie Benzylalkohol, p-Chlor-m-cresol, Solbrol, Polyäthylenglykol und Kollidon. Diesen Versuchen war jedoch auch kein Erfolg beschieden.

Überraschenderweise konnte nun ein auf der ICHS-Referenzmethode basierendes, für die Verwendung in Kunststoff-Einweg-Küvetten geeignetes Reagenz gefunden werden, das die geforderte Langzeitstabilität besitzt. Die erfindungsgemässe Reaktionslösung zeichnet sich dadurch aus, dass der pH-Wert mit Hilfe der Puffersubstanz innerhalb eines engen Bereiches von 7,1 bis 7,3 eigestellt ist und die Kaliumcyanidkonzentration 90 bis 120 mg/l (1,38 bis 1,85 mmol/l) beträgt. Die Erfindung betrifft auch die Verwendung dieser Reaktionslösung zur Bestimmung des Hämoglobins mit Hilfe von Kunststoff-Einweg-Messküvetten.

Bei Verwendung dieses Reagenzes in Polystyrol-Fertig-Küvetten bildet sich nach kurzer Zeit ein quasistabiler Zustand aus, so dass eine Haltbarkeit in Polystyrol-Fertig-Küvetten von mindestens 12 Monaten erreicht wird.

Das Reagenz entspricht im stabilen Zustand der ICSH-Referenzmethode. Hinsichtlich der Umwandlungsgeschwindigkeit des Hämoglobins in Hämiglobincyanid (Reaktionsgeschwindigkeit) bestehen keine Unterschiede zwischen beiden Reagenzien. Die Ergebnisse von 431 Hämoglobinbestimmungen, bei denen der Hämoglobingehalt jeweils einer Blutprobe sowohl mit der ICSH-Referenzmethode als auch mit dem erfindungsgemässen Reagenz bestimmt wurde, zeigen eine sehr gute Übereinstimmung. Als Mittelwert wurden 15,0 g/100 ml Hämoglobin mit der ICSH-Referenzmethode gefunden. Mit dem erfindungsgemässen Reagenz wurde ein Mittelwert von 14,8 g/100 ml erhalten.

Die gefundenen Wertepaare wurden korreliert. Als Korrelationsgerade wurde berechnet: x = 1,0139 · y − 0,0333, wobei x den mit der ICSH-Referenzmethode und y den mit dem erfindungsgemässen Reagenz bestimmten Werten entspricht. Der Korrelationskoeffizient wurde mit r = 0,9934 berechnet.

*Ausführungsbeispiel*

Zur Abfüllung in eine Polystyrol-Einweg-Messküvette wurde ein Hämoglobinreagenz folgender Zusammensetzung hergestellt:
1,54 mmol/l Kaliumcyanid
0,6 mmol/l Kaliumhexacyanoferrat (III)
2,5 mmol/l Kaliumphosphatpuffer pH 7,1 bis 7,3
0,05 % Detergenz (z.B. Saponin)

Hieraus wurden unter Verwendung von destilliertem Wasser als Lösungsmittel 1,25 ml Lösung gewonnen. Zur Vermeidung von Trübungen können dieser Lösung gegebenenfalls noch geringe Mengen von NaCl (ca. 1,5 mmol/l) zugesetzt werden.

*Literatur*

1. International Commitee for Standardization in Haematology: „Recommendations for Haemoglobinometry in Human Blood", *in* „British Journal of Haematology", 13 (1967), Suppl. 71-75.

2. W.G. Zijlstra und E.J. van Kampen: „Standardization of Haemoglobinometry. I. The Extinction Coefficient of Haemiglobincyanide", *in* „Clinica Chimica Acta", 5 (1960) 719-726.

3. E.J. van Kampen und W.G. Zijlstra: „Standardization of Haemoglobinometry. II. The Haemiglobincyanide Method", in „Clinica Chimica Acta", 6 (1961), 538-544.

4. „Bestimmung des Hämoglobingehaltes im Blut", Vornorm DIN 58 931, Blatt 1, November 1970.

**Patentansprüche**

1. Kunststoff-Einweg-Messküvette für die Hämoglobinbestimmung enthaltend eine Reaktionslösung aus Kaliumcyanid, Kaliumhexacyanoferrat (III), einer Puffersubstanz und einem Detergenz, dadurch gekennzeichnet, dass der pH-Wert der Lösung auf einen Wert im Bereich von 7,1 bis 7,3 eingestellt ist und die Kaliumcyanidkonzentration 90 bis 120 mg/l (1,38 bis 1,85 mmol/l) beträgt.

2. Messküvette nach Anspruch 1, dadurch gekennzeichnet, dass sie aus Polystyrol besteht.

3. Verwendung einer Reaktionslösung aus 90 bis 120 mg/l (1,38 bis 1,85 mmol/l) Kaliumcyanid mit einem durch Puffersubstanzen eingestellten pH-Wert im Bereich von 7,1 bis 7,3 unter Zusatz von Kaliumhexacyanoferrat (III) und einem Detergenz zur Bestimmung des Hämoglobins mit Hilfe von Kunststoff-Einweg-Küvetten.

## Claims

1. Plastic throw-away measuring cell for the determination of haemoglobin, containing a reaction solution of potassium cyanide, potassium hexacyanoferrate-(III), a buffer substance and a detergent, characterised in that the pH value of the solution is adjusted to a value in the range from 7.1 to 7.3 and the potassium cyanide concentration is 90 to 120 mg/l (1.38 to 1.85 mmol/l).

2. Measuring cell according to Claim 1, characterised in that it consists of polystyrene.

3. Use of a reaction solution of 90 to 120 mg/l (1.38 to 1.85 mmol/l) of potassium cyanide, having a pH value, adjusted by means of buffer substances, in the range from 7.1 to 7.3, with the addition of potassium hexacyanoferrate-(III) and a detergent, for the determination of haemoglobin with the aid of plastic throw-away measuring cells.

## Revendications

1. Cuvette de mesure à une voie en matière plastique pour la détermination de l'hemoglobine, contenant une solution de réaction de cyanure de potassium, d'hexacyanoferrate (III) de potassium d'une substance-tampon et d'un détergent, caractérisée en ce que la valeur de pH de la solution est réglée à une valeur dans l'intervalle de 7,1 à 7,3, et en ce que la concentration en cyanure de potassium s'élève à 90 à 120 mg/l (1,38-1,85 mmol/l).

2. Cuvette de mesure selon la revendication 1, caractérisée en ce qu'elle consiste en du polystyrène.

3. Utilisation d'une solution de réaction à partir de 90 à 120 mg/l (1,38-1,85 mmol/l) de cyanure de potassium avec une valeur de pH, réglée par des substances-tampons, dans l'intervalle de 7,1 à 7,3, avec addition d'hexacyanoferrate (III) de potassium et d'un détergent pour la détermination de l'hémoglobine à l'aide de cuvettes à une voie en matière plastique.